# EUROPEAN PATENT SPECIFICATION

(11) **EP 4 497 514 B1**
(45) Date of publication and mention of the grant of the patent: **15.04.2026**
(21) Application number: 23187138.5
(22) Date of filing: 24.07.2023
(51) Int. Cl.: B08B 3/02, A47L 15/42, A61B 90/70, A61L 2/07, F28C 3/06, F28C 3/08

(54) **THERMAL CLEANING AND DISINFECTING DEVICE**
THERMISCHE REINIGUNGS- UND DESINFEKTIONSVORRICHTUNG
DISPOSITIF DE NETTOYAGE ET DE DÉSINFECTION THERMIQUE

(43) Date of publication of application: 29.01.2025
(73) Proprietor: W & H Sterilization S.r.l., 24060 Brusaporto (BG) (IT)
(72) Inventor: Miranda, Marco, 24060 Brusaporto (IT); Bettineschi, Daniele, 24069 Trescore Balneario (IT); Crotti, Alex, 24011 Almè (IT); Magno, Marin Luigi, 24031 Almenno San Salvatore (IT); Maier, Klaus, 5020 Salzburg (AT)
(74) Representative: Benda, Ralf

(56) References cited:
- EP-A1- 3 133 974
- WO-A1-2022/113141
- US-A- 4 732 712
- US-A- 4 773 827
- US-A1- 2007 104 608

## Description

The present invention relates to a thermal cleaning and disinfecting device for cleaning and disinfecting medical, surgical, dental and/ or veterinary items as well as household items, such as dishes, cutlery etc.

Such a thermal cleaning and disinfecting device, often referred to as thermal washer disinfector (TWD) in the medical field or as dish washer in the domestic field, is well known and widely used. Usually, the comprise a washing chamber for receiving items to be cleaned and disinfected; a pump for circulating a cleaning and disinfecting liquid; orifices arranged in the washing chamber for applying the cleaning and disinfecting liquid to the items to be cleaned and disinfected; a control device; and a heating system for heating the cleaning and disinfecting liquid.

Some thermal cleaning and disinfecting devices known from the prior art in addition supply not only hot water but also steam to the washing chamber and the items in the washing chamber, see for example patent application US 2004/261824 A1. These thermal cleaning and disinfecting devices have a steam generator and openings which deliver the steam to the washing chamber. The steam is intended to clean heavily soiled items and to promote disinfection of the items.

Document EP 3 133 974 A1 discloses, in accordance with the preamble of claim 1, a washer/disinfector having a washing chamber to receive articles to be washed, a sump at the bottom of the washing chamber and a fluid-heating system with a steam generator. A steam line supplies steam from the steam generator to a heat exchanger which extends through the sump of the washer/disinfector to heat the liquid in the sump. Through an exhaust port of the fluid-heating system residual steam from the heat exchanger is exhausted into the washing chamber.

Document US 2007/104608 A1 discloses an automatic cleaning and disinfecting machine for containers containing human excreta. The machine comprises a chamber to accommodate the containers to be disinfected and a steam generator which supplies steam to the chamber for the heat disinfection of the containers in the chamber. A corresponding machine for treating objects, in particular for washing and sanitizing, is described in document WO 2022/113141: in a sanitizing step the objects are treated with steam which is generated in a steam generator and supplied to the objects via a feed.

Document US 4,773,827 discloses a liquid heating apparatus capable of using residual low pressure steam from processing operations of industrial plants to heat a liquid, particularly where the liquid is at a higher pressure than the steam. Similarly, document US 4,732,712 describes an apparatus for controlling the temperature of a supply of water by selective injection of steam in industrial settings like food processing and manufacturing.

It is an object of the present application to provide a thermal cleaning and disinfecting device having an improved heating regime for the cleaning and disinfecting liquid. In particular, it is an object to speed up the heating of the cleaning and disinfecting liquid, so that an overall time for cleaning and disinfecting of the items by the thermal cleaning and disinfecting device can be reduced.

These objects are achieved by a thermal cleaning and disinfecting device for cleaning and disinfecting medical, surgical, dental, veterinary as well as household items according to Claim 1 and by a method for heating a cleaning and disinfecting liquid in a thermal cleaning and disinfecting device according to Claim 15. Particularly advantageous embodiments are specified in the dependent claims.

A thermal cleaning and disinfecting device for cleaning and disinfecting medical (including surgical, dental and veterinary) as well as household items comprises a washing chamber for receiving items to be cleaned and disinfected; a circulating pump for circulating a cleaning and disinfecting liquid; a plurality of orifices arranged in the washing chamber for applying the cleaning and disinfecting liquid to the items to be cleaned and disinfected; a control device; and a heating system for heating the cleaning and disinfecting liquid and connecting to the plurality of orifices. The heating system comprises a steam generator and a heat transfer device, wherein the heat transfer device is configured to receive steam generated by the steam generator and the cleaning and disinfecting liquid circulated by the circulating pump. The heat transfer device comprises a mixing section in which the steam and the cleaning and disinfecting liquid mix, such that steam condensates and transfers heat to the cleaning and disinfecting liquid. Due to the heat transfer the temperature of the cleaning and disinfecting liquid raises, preferably to 50°C or more, especially preferably to about 90°C. The heat transfer device, in particular the mixing section, comprises an outlet which connects to the plurality of orifices to supply the cleaning and disinfecting liquid to the plurality of orifices.

Due to the provision of the steam generator and the heat transfer device, in particular the mixing section, in which the steam and the cleaning and disinfecting liquid mix or intermix and the cleaning and disinfecting liquid is heated by the steam, the heating of the cleaning and disinfecting liquid is accelerated and thus the overall time for cleaning and disinfecting of the items by the thermal cleaning and disinfecting device is reduced. Also, the temperature of the cleaning and disinfecting liquid is kept at a determined level by mixing steam and the cleaning and disinfecting liquid in the heat transfer device.

According to an embodiment the steam generator is electrically powered. Preferably, the steam generator comprises an electrically powered heating element, such as a heating rod or coil. This advantageously makes it possible to easily control the production of the steam, e.g., adapt the power supplied to the steam generator to the amount of liquid or water conveyed to the steam generator.

According to an embodiment the steam generator comprises a flow boiling steam generator. Preferably, liquid or water is, in particular continuously, supplied by a feeding pump to the flow boiling steam generator, and passes through it, such that it evaporates continuously along the passage through the steam generator. A flow boiling steam generator provides a continuous flow of steam, in particular to the heat transfer device, so that advantageously the cleaning and disinfecting liquid can be continuously heated by steam and the pressure between the heat transfer device and the steam generator is automatically balanced.

According to another embodiment the steam generator comprises a pool boiling steam generator. Preferably, liquid or water is supplied, in particular intermittently, by a feeding pump to a pool or container where it evaporates. The steam is then, in particular intermittently, conveyed to the heat transfer device. A pool boiling steam generator is easier to design compared to a flow boiling steam generator and can thus advantageously be easier integrated into a thermal cleaning and disinfecting device.

According to an embodiment the thermal cleaning and disinfecting device comprises a feeding pump which is configured to supply liquid or water to the steam generator. Preferably, the feeding pump is arranged upstream of the steam generator and/ or between the steam generator and a liquid or water source. Preferably, the feeding pump is communicatively connected to and/ or controlled by the control device, such that the feed rate of liquid or water to the steam generator and thus the amount of generated steam can be controlled and varied by the control device. For example, the control device is configured to control the feeding pump, such that the feed rate of the feeding pump depends on at least one of: the temperature of the cleaning and disinfecting liquid; the quantity or type of items in the washing chamber; an operating program of the thermal cleaning and disinfecting device. Varying the feed rate of liquid or water to the steam generator makes it possible to produce only the amount of steam, which is needed, which advantageously saves time and energy.

Alternatively, and/ or in addition to the feeding pump a feeding valve is arranged upstream of the steam generator and/ or between the steam generator and a liquid or water source. Preferably, the feeding valve is configured to control the supply of liquid or water from the source to the steam generator. Preferably, the feeding valve comprises a control valve. Preferably, the feeding valve is communicatively connected to and/ or controlled by the control device, such that the feed rate of liquid or water to the steam generator and thus the generated amount of steam can be controlled and varied by the control device. For example, the control device is configured to control the feeding valve, such that the supply of water or liquid depends on at least one of: the temperature of the cleaning and disinfecting liquid; the quantity or type of items in the washing chamber; an operating program of the thermal cleaning and disinfecting device. Varying the feed rate of liquid or water to the steam generator makes it possible to produce only the amount of steam, which is needed, which advantageously saves time and energy.

According to an embodiment a valve is provided to control the flow or supply of steam from the steam generator to the heat transfer device. The valve is also referred to as steam valve in the following. Preferably, the steam valve is a control valve which his controllable by the control device. Preferably, the steam valve comprises a check valve. Preferably, the valve is configured as part of the steam generator and/ or implemented in the steam generator. Preferably, the valve is configured as part of the heat transfer device and/ or implemented in the heat transfer device. Preferably, the valve is arranged between the steam generator and the heat transfer device, in particular downstream of the steam generator and/ or upstream of the heat transfer device. Preferably, the valve is arranged in a pipe connecting the steam generator and the heat transfer device. Providing a valve, in particular a control valve, makes it possible to vary the feed rate of steam to the heat transfer device and thus to deliver steam only when it is needed and/ or only the amount of steam which is needed, which advantageously saves time and energy. Providing a steam valve also has the advantage that the pressure of the steam can be controlled, which is important to achieve a good mixture of the steam and the cleaning and disinfecting liquid in the heat transfer device or mixing section, in particular when the steam generator comprises a pool boiling steam generator.

Alternatively, or in addition to the valve a throttle or reduction, in particular of the pipe's inner diameter, in the pipe connecting the steam generator and the heat transfer device can be provided. The throttle or reduction may help to raise the pressure in the steam generator and/ or the velocity of the steam, which is advantageous to obtain a good mixture of steam and the cleaning and disinfecting liquid in the heat transfer device.

According to an embodiment the generation of steam and/ or the supply or feed rate of the steam to the heat transfer device may be varied or controlled by the control device, in particular to set the temperature of the cleaning and disinfecting liquid and/ or depending on the temperature of the cleaning and disinfecting liquid. Accordingly, the control device is configured to control the amount of steam transferred to the heat transfer device. Control of the feed rate by the control device advantageously provides an optimized energy management (system), such that steam is produced and/ or supplied to the heat transfer device only when it is required. According to an embodiment the control device is configured to stop the generation and/ or supply of steam to the heat transfer device, so that no steam is generated, supplied to or enters the heat transfer device. Thus advantageously, during washing or rinsing steps in which no warm cleaning and disinfecting liquid is needed no energy for producing or supplying steam is consumed. According to another embodiment the control device is configured to control the supply of steam to the heat transfer device but is not configured to fully stop the generation or delivery of steam to the heat transfer device, so that at least a small amount of steam is constantly produced and/ or delivered towards the heat transfer device. This may advantageously even more reduce the overall time for cleaning and disinfecting of the items, since steam can be produced and/ or supplied to the heat transfer device during washing or rinsing steps in which no warm cleaning and disinfecting liquid is needed.

According to an embodiment the control of the generation of steam and/ or the supply of the steam to the heat transfer device by the control device is achieved by an actor which is controllable by the control device. Preferably, the controllable actor is configured such that it acts on the supply or flow of liquid or water that is supplied to the steam generator or on the generated steam. Preferably, the controllable actor comprises a control valve to control the flow of steam from the steam generator to the heat transfer device, see above, and/ or by a controllable feeding pump and/ or feeding valve to control the feed of liquid or water to the steam generator, see above. Preferably, the thermal cleaning and disinfecting device may comprise a regulating or control circuit to regulate or control generation of steam and/ or the feed rate of the steam to the heat transfer device and/ or the temperature of the cleaning and disinfecting liquid. The regulating or control circuit preferably comprises the control device, at least one actor controllable by the control device, e.g., the feeding pump, the feeding valve or the control valve to control the flow of steam, and a temperature sensor which is configured to determine the temperature of the cleaning and disinfecting liquid and will be described in the following.

The heat transfer device is configured to receive steam generated by the steam generator and the cleaning and disinfecting liquid circulated by the circulating pump. According to an embodiment, the heat transfer device is configured as or comprises a mixing device. Preferably, the heat transfer device comprises a pipe or tube. Preferably, the heat transfer device comprises an inner cavity or hollow space. Preferably, the heat transfer device is configured such that the steam and the cleaning and disinfecting liquid physically contact one another and/ or mix and/ or intermix, in particular in the inner cavity of the heat transfer device and/ or in the inner cavity of a mixing section of the heat transfer device. These features advantageously promote or support the heat transfer from the steam to the cleaning and disinfecting liquid.

According to an embodiment the heat transfer device or the length of the heat transfer device, in particular the length of the mixing section or pipe, is configured such, that the entire amount of steam received by the heat transfer device condensates within the heat transfer device. Preferably, the heat transfer device is configured such that no steam exits the heat transfer device and/ or that no steam enters the washing chamber. Preferably, the heat transfer device is configured such that the mixture of the steam and the cleaning and disinfecting liquid and/ or the, particularly complete, condensation of the steam takes place in the heat transfer device and/ or before the cleaning and disinfecting liquid enters the washing chamber and/ or before the cleaning and disinfecting liquid leaves the heat transfer device. Preferably, the heat transfer device and/ or the thermal cleaning and disinfecting device is/ are configured such that no steam is delivered by the plurality of orifices into the washing chamber. These features advantageously guarantee a maximum heat transfer from the steam to the cleaning and disinfecting liquid.

The heat transfer device and the washing chamber are different, distinct and/ or separate devices. According to an embodiment, at least a portion of or the entire heat transfer device is arranged outside the washing chamber. Thus, the steam generator and the heat transfer device can be placed close to one another, so that advantageously loss of heat of the steam on its way from the steam generator to the heat transfer device is reduced. According to another embodiment, at least a portion of or the entire heat transfer device is arranged within the washing chamber, which advantageously saves space in the thermal cleaning and disinfecting device.

According to an embodiment the heat transfer device comprises a pipe or tube. Preferably, the heat transfer device comprises a straight pipe which in particular extends along a longitudinal axis. Preferably, the straight pipe has a length of at least 6 cm, in particular of more than 10 cm. Due to its small width a straight pipe can easily be arranged in the thermal cleaning and disinfecting device. Particularly preferred, the straight pipe has a length between 25 cm and 50 cm, or between 30 cm and 40 cm, in particular about 35 cm. According to another embodiment the heat transfer device, in particular the mixing section, comprises a bent or curved pipe or a pipe having a spiral section or a spiral shape. Providing a pipe having at least one bending or a spiral shape may advantageously further promote the mixing and heat transfer between the steam and the cleaning and disinfecting liquid.

According to another embodiment the heat transfer device comprises a disk or disk-like shape or a funnel or funnel-like shape with a hollow interior which is configured as the mixing section. In particular, the hollow interior is comprised by a body of the disk- or funnel shaped heat transfer device. A disk- or funnel shaped heat transfer device is very compact and is thus advantageously suited for arrangement inside the washing chamber. Preferably, inlets for the steam and the cleaning and disinfecting liquid meet the disk- or funnel shaped heat transfer device at the body and/ or a side wall thereof. Preferably, inlets for the steam and the cleaning and disinfecting liquid enter the side wall at different positions. In particular at least one of the inlets enters the side wall tangentially. Preferably, the disk- or funnel shaped heat transfer device is configured such that at least one of the steam and the cleaning and disinfecting liquid form(s) a vortex when it/they flow(s) in or through the heat transfer device. Both, an inlet entering the side wall tangentially and the shape which causes a vortex advantageously further promote the mixing and heat transfer between the steam and the cleaning and disinfecting liquid. Preferably, an outlet of the disk- or funnel shaped heat transfer device, through which the heated cleaning and disinfecting liquid exits towards the washing chamber, extends along an axis which is disposed at an angle to a plane in which at least a part, in particular the body, of the disk- or funnel shaped heat transfer device and/ or the inlets for the steam and the cleaning and disinfecting liquid are arranged, in particular at angle of about 90°.

According to yet another embodiment the heat transfer device comprises at least two of the shapes described in the foregoing, i.e., a straight pipe; a bent pipe; a spiral pipe; a heat transfer device having a disk or disk-like shape; a heat transfer device having a funnel or funnel-like shape. For example, a heat transfer device has a first section which comprises a straight pipe and a second section comprising a disk-like shape. Of course, any other combination of two or more of these shapes is also possible. Such combination of shapes further promotes the mixing and heat transfer between the steam and the cleaning and disinfecting liquid, while providing a compact shape.

According to an embodiment the heat transfer device comprises a first inlet for the cleaning and disinfecting liquid and a second inlet for the steam, wherein the first inlet and the second inlet are distinct inlets which are separated from one another. Preferably, the first inlet connects to the circulating pump for circulating a cleaning and disinfecting liquid and/ or the washing chamber, and the second inlet connects to the steam generator. Preferably, the first and second inlets are arranged at a first end portion of the heat transfer device or form the first end portion, in particular the first end portion of the pipe of the heat transfer device. Preferably, the heat transfer device or the pipe has a first branch and a second branch, wherein the first branch connects to the first inlet and the second branch connects to the second inlet. Preferably, the first and second branches form the first end portion of the heat transfer device. Preferably, the first and second branch are arranged such that they form a Y or a Y-shaped first end portion of the heat transfer device. These features ensure reliable uptake of the steam and the cleaning and disinfecting liquid into the heat transfer device.

According to an embodiment the outlet of the heat transfer device comprises a single outlet opening which connects to orifices of the plurality of orifices which are configured to receive the cleaning and disinfecting liquid heated by the steam and to apply the cleaning and disinfecting liquid to the items to be cleaned and disinfected. Preferably, the outlet of the heat transfer device and the plurality of orifices are connected by one or more pipes. Preferably, orifices of the plurality of orifices are arranged on a rotatable arm in the wash chamber, so that the outlet of the heat transfer device connects to orifices on the rotatable arm. Preferably, orifices of the plurality of orifices are arranged on a wall of the wash chamber, so that the outlet of the heat transfer device connects to orifices on the wall. These features ensure a reliable distribution of the cleaning and disinfecting liquid heated by the steam to orifices of the plurality of orifices which are disposed on different elements or areas of the thermal cleaning and disinfecting device.

According to another embodiment the outlet of the heat transfer device comprises a single outlet opening which connects to orifices of the plurality of orifices via a sump or collecting tank that is arranged at the bottom of the washing chamber. Preferably, the single outlet opening first connects to the collecting tank which connects to the orifices. Preferably, the outlet of the heat transfer device and the collecting tank connect by one or more pipes. According to yet another embodiment the outlet of the heat transfer device comprises a plurality of outlet openings. Preferably, one opening of these plurality of openings connects directly to orifices of the plurality of orifices while another opening of the plurality of openings directly connects to the collecting tank and/ or first connects to the collecting tank which connects to the orifices. The connection of the heat transfer device to the sump or collecting tank advantageously decreases the temperature loss of the cleaning and disinfecting liquid in the sump or collecting tank, in particular when in a step of a cleaning or disinfecting procedure no cleaning and disinfecting liquid is conveyed to the washing chamber or plurality of orifices.

According to an embodiment the outlet of the heat transfer device, in particular the single outlet opening, is arranged at a second end of the heat transfer device and/ or forms the second end. Preferably, the second end is opposite the first end of the heat transfer device. Preferably, the heat transfer device comprises a straight pipe with a first end having at least one of the first inlet or second inlet and a second end having the outlet and which is opposite the first end. These features provide a compact heat transfer device which can be easily accommodated in the thermal cleaning and disinfecting device.

According to an embodiment the heat transfer device comprises a mixing section and a connecting section. Preferably, the connecting section comprises the first branch, the first inlet, the second branch and the second inlet. Preferably, the mixing section and the connecting section are formed in one-piece and/ or form a one-piece, in particular straight, pipe comprised by the heat transfer device. Preferably, relative to the longitudinal axis of the heat transfer device or pipe, the mixing section is longer than the connecting section. These features provide a compact heat transfer device which can be easily integrated into the thermal cleaning and disinfecting device.

According to an embodiment the heat transfer device, in particular the mixing section, comprises at least one geometric shape which is configured to promote the intermixing of the steam and the cleaning and disinfecting liquid. Preferably, the at least one geometric shape is arranged inside the heat transfer device, in particular the mixing section or pipe. Preferably, the at least one geometric shape is arranged only in a portion of the mixing section or pipe. Preferably, the at least one geometric shape projects from an inner surface of a wall of the heat transfer device, in particular of the mixing section or pipe. Preferably, the at least one geometric shape projects into the cavity of the mixing section or pipe. Preferably, the at least one geometric shape comprises a projection. Preferably, the at least one geometric shape is configured to cause or promote turbulent flow of the steam and/ or the cleaning and disinfecting liquid. These features advantageously foster heat transfer from the steam to the cleaning and disinfecting liquid.

According to an embodiment the thermal cleaning and disinfecting device comprises at least one temperature sensor which is configured to determine the temperature of the cleaning and disinfecting liquid. Preferably, the temperature sensor is part of the regulating or control circuit to regulate or control generation of steam and/ or the feed rate of the steam to the heat transfer device and/ or the temperature of the cleaning and disinfecting liquid, see above. Preferably, the temperature sensor is configured to determine the temperature of the cleaning and disinfecting liquid and to send a temperature signal indicative of the temperature of the cleaning and disinfecting liquid to the control device. Preferably, the temperature sensor is communicatively connected to the control device. Preferably, the control device controls the generation of steam and/ or supply of steam to the heat transfer device based on the temperature signal indicative of the determined temperature of the cleaning and disinfecting liquid. The provision of a temperature sensor advantageously supports and improves control processes of the thermal cleaning and disinfecting device, in particular the regulation or control of generation of steam and/ or the feed rate of the steam.

According to an embodiment the temperature sensor is arranged at the bottom of the washing chamber to measure the temperature of the cleaning and disinfecting liquid at the bottom of the washing chamber. Preferably, a sump or collecting tank is arranged at the bottom of the washing chamber or is formed as part of the bottom of the washing chamber. Preferably, the temperature sensor is arranged in or at the sump or collecting tank, so that the temperature sensor provides the temperature of the cleaning and disinfecting liquid in the collecting chamber. Since the temperature of the cleaning and disinfecting liquid in the collecting chamber is low or lowest during circulation of the cleaning and disinfecting liquid, the control of the generation or supply of steam is advantageously based on this low temperature.

According to another embodiment a temperature sensor or in addition to the temperature sensor described in the foregoing, which may be a first temperature sensor, a second temperature sensor is arranged downstream and/ or next to heat transfer device, so that the (second) temperature sensor provides the temperature of the cleaning and disinfecting liquid after the heat transfer device. Preferably, the temperature sensor is arranged in or at a pipe connecting the heat transfer device to orifices of the plurality of orifices. Preferably, the temperature sensor is arranged in or adjacent to the outlet of the heat transfer device. Preferably, the (second) temperature sensor is part of the regulating or control circuit to regulate or control generation of steam and/ or the feed rate of the steam to the heat transfer device and/ or the temperature of the cleaning and disinfecting liquid as described above for the first temperature sensor. Locating the (second) temperature sensor downstream to the heat transfer device makes it advantageously possible to regulate the generation and/ or the supply of steam and/ or the temperature of the cleaning and disinfecting liquid and/ or to establish a corresponding control loop or regulating circuit.

According to an embodiment the control device is configured to control the supply of a cleaning or disinfecting agent, for example a detergent, to the cleaning and disinfecting liquid. Preferably, the cleaning or disinfecting agent is liquid and is arranged in a reservoir. Preferably, the reservoir connects to a pipe in the thermal cleaning and disinfecting device which conducts the cleaning and disinfecting liquid and/ or to the washing chamber, so that the cleaning or disinfecting agent can be added to the cleaning and disinfecting liquid. Preferably, the thermal cleaning and disinfecting device comprises an agent actuator, e.g. an agent pump, which is configured to deliver cleaning or disinfecting agent to the pipe, washing chamber and/ or cleaning and disinfecting liquid. Preferably, the agent pump comprises a controllable pump, which in particular is controllable by the control device, so that advantageously the amount of added cleaning or disinfecting agent can be varied. Alternatively or in addition to the agent pump, the agent actuator may comprise an agent valve, in particular controllable by the control device, which is configured to control the delivery of the cleaning or disinfecting agent from the reservoir to the cleaning and disinfecting liquid.

According to an embodiment the thermal cleaning and disinfecting device comprises a sensor for determining the concentration of the cleaning or disinfecting agent in the cleaning and disinfecting liquid. Preferably, the sensor is arranged at or in a pipe in the thermal cleaning and disinfecting device which conducts the cleaning and disinfecting liquid and/ or at or in the washing chamber, particularly at the bottom, and/ or at or in the sump of the washing chamber. Preferably, the sensor comprises a conductivity sensor. Preferably, the sensor is communicatively connected to the control device. Preferably, the sensor for determining the concentration of the cleaning or disinfecting agent is configured to send a concentration signal indicative of the determined concentration of the cleaning or disinfecting agent in the cleaning and disinfecting liquid to the control device. Preferably, the control device is configured to control the supply of cleaning or disinfecting agent to the cleaning and disinfecting liquid based on the concentration signal. These features advantageously make it possible to counteract the dilution of the cleaning or disinfecting agent in the cleaning and disinfecting liquid and in particular to keep its concentration constant, since due to the adding of steam to the cleaning and disinfecting liquid the concentration of the cleaning or disinfecting agent decreases.

According to another embodiment the sensor for determining the concentration of the cleaning or disinfecting agent is arranged near and downstream of the reservoir for the cleaning and disinfecting agent. This makes it advantageously possible to regulate the delivery of the cleaning or disinfecting agent to the cleaning and disinfecting liquid and/ or to establish a corresponding control loop or regulating circuit for the delivery of the agent.

In addition to the elements already described in the foregoing, the thermal cleaning and disinfecting device may comprise one or more of the following elements, such as:
- an outer housing which forms an outer casing of the thermal cleaning and disinfecting device and/ or surrounds the other components of the thermal cleaning and disinfecting device, in particular one or more or all of the following components: the washing chamber, the control device, the heating system with the steam generator and a heat transfer device; one or more pumps, like the feeding pump, the agent pump and the circulating pump for circulating the cleaning and disinfecting liquid; one or more containers, in particular for water for the steam generator, a cleaning or disinfecting agent, e.g. a detergent, a water softener, regenerating salt, etc., electric lines, pipes and other elements of the thermal cleaning and disinfecting device;
- a door for opening and closing an opening in the outer housing which connects to the washing chamber, so that the items can be put in and out of the washing chamber;
- a control panel which is operable for a user and communicatively connected to the control device, so that a user can select operating programs or set operational parameters;
- a display which is configured to display operating programs, alarms, errors, the current operating status of the thermal cleaning and disinfecting device, in particular whether the steam generator is producing steam or not and/ or whether steam is fed to the heat transfer device and mixing section, respectively, or not and/ or whether the cleaning and disinfecting liquid is currently heated up by steam or not, etc.
- a connector to the mains for supplying power to the thermal cleaning and disinfecting device and/ or a connector to an external water supply.

According to an embodiment at least one orifice of the plurality of orifices arranged in the washing chamber for applying the cleaning and disinfecting liquid to the items to be cleaned and disinfected may comprise a nozzle. At least one orifice of the plurality of orifices arranged in the washing chamber may be arranged on an arm, in particular a rotating arm, arranged in the washing chamber. At least one orifice of the plurality of orifices arranged in the washing chamber may be disposed in a wall of the washing chamber, in particular in a side wall or the top wall.

According to an embodiment all orifices of the plurality of orifices connect to the heat transfer device, so that in particular all orifices are supplied with cleaning and disinfecting liquid or water heated by steam and/ or having the same temperature, so that advantageously a user can arrange the items to be washed and disinfected at any place in the washing chamber, since all items are sprayed with cleaning and disinfecting liquid of the same temperature. According to another embodiment only one or some of the orifices of the plurality of orifices connect to the heat transfer device, so that in particular not all orifices are supplied with cleaning and disinfecting liquid heated by steam and/ or having the same temperature. This may be advantageous if the thermal cleaning and disinfecting device has areas or levels with different heat regimes, so that for example, a user can place items which should be spray or treated with cleaning and disinfecting liquid that has been heated by steam and thus having a higher temperature to a certain area.

According to an embodiment a method for heating a cleaning and disinfecting liquid in a thermal cleaning and disinfecting device, in particular in a thermal cleaning and disinfecting device described in the foregoing, comprises: providing a heating system comprising a steam generator and a heat transfer device; supplying steam generated by the steam generator and cleaning and disinfecting liquid to the heat transfer device; mixing or intermixing the steam and the cleaning and disinfecting liquid in a mixing section of the heating system, such that the steam condensates and transfers heat to the cleaning and disinfecting liquid.

According to an embodiment, the generation of steam in the steam generator and/ or supply of the steam to the heat transfer device is controlled by the control device. For example, the control device controls the feeding valve and/ or the feeding pump and/ or the steam valve to control the flow of steam from the steam generator to the heat transfer device depending on at least one of: the temperature of the cleaning and disinfecting liquid; the quantity or type of items in the washing chamber; an operating program of the thermal cleaning and disinfecting device.

The method for heating a cleaning and disinfecting liquid may comprise one or more of the features and elements described in the foregoing. Thus, to avoid repetition, reference is made to the foregoing. Accordingly, any feature that is described in the foregoing may also be incorporated into said method.

Preferred embodiments will be described below with reference to the following drawings:
Figure 1 shows a schematic diagram of a thermal cleaning and disinfecting device with a the heating system having a steam generator and a heat transfer device;
Figure 2 shows a first embodiment of a heat transfer device configured to mix steam and a cleaning and disinfecting liquid to raise the temperature of the cleaning and disinfecting liquid;
Figure 3 shows a second embodiment of a heat transfer device configured to mix steam and a cleaning and disinfecting liquid to raise the temperature of the cleaning and disinfecting liquid;
Figure 4 shows a third embodiment of a heat transfer device configured to mix steam and a cleaning and disinfecting liquid to raise the temperature of the cleaning and disinfecting liquid;
Figure 5 shows a side view of the heat transfer device of Fig. 3 showing the outlet through which the heated cleaning and disinfecting liquid leaves the heat transfer device;
Figure 6 shows an alternative embodiment of a heating system of a thermal cleaning and disinfecting device;
Figure 7 shows a further alternative embodiment of a heating system of a thermal cleaning and disinfecting device.

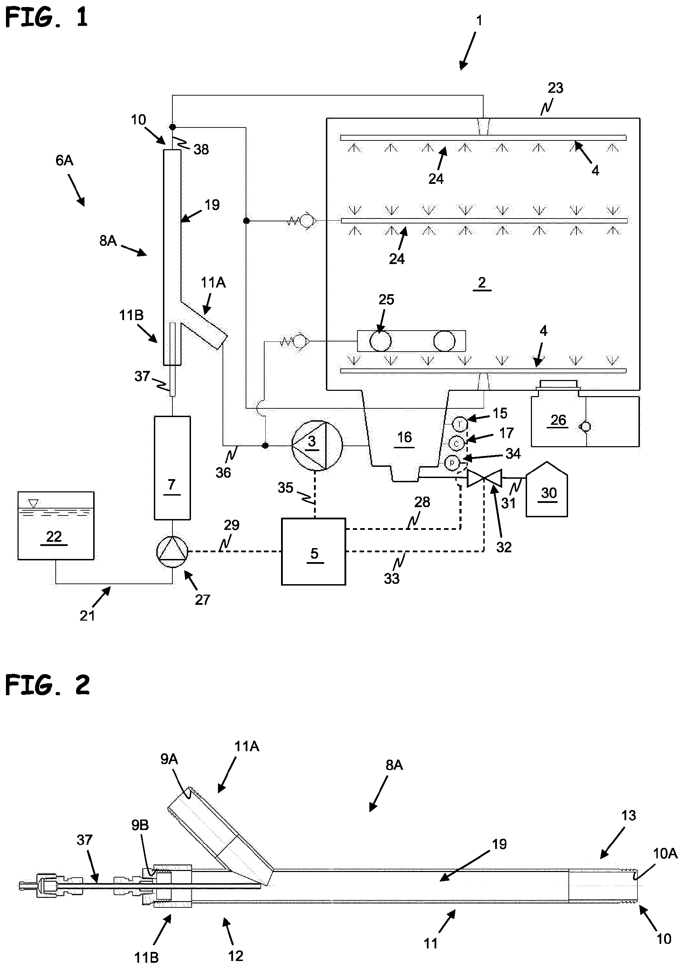

Figure 1 shows a thermal cleaning and disinfecting device 1 for cleaning and disinfecting medical, surgical, dental, veterinary as well as household items. Thermal cleaning and disinfecting device 1 comprises a washing chamber 2 for receiving the items to be cleaned and disinfected, a control device 5, a heating system 6A, a circulating pump 3 for circulating a cleaning and disinfecting liquid, and a connection 21 to a liquid source 22, in particular a water source, to supply heating system 6A with liquid or water. The liquid source 22 may comprise a tank or the public water supply, while connection 21 may comprise a pipe.

Washing chamber 2 comprises an enclosure 23 which accommodates a plurality of orifices 4 for applying the cleaning and disinfecting liquid to the items to be cleaned and disinfected. At least some of the orifices of the plurality of orifices 4 are arranged on rotating arms 24 within enclosure 23. Some of the orifices of the plurality of orifices 4 may be arranged on an inner surface of enclosure 23. Preferably, washing chamber 2 in addition comprises at least one connection port 25 to which an item, e.g., a handpiece, to be cleaned and disinfected internally can be coupled. A collecting tank or sump 16 is provided at the bottom of the washing chamber 2. After ejection of the cleaning and disinfecting liquid through the plurality of orifices 4 the cleaning and disinfecting liquid collects in collecting tank 16. A reservoir 26 for an additive, e.g., a water softener or regenerating salt, is connected to washing chamber 2, so that the additive can be delivered to washing chamber 2 and/ or the cleaning and disinfecting liquid.

Circulating pump 3 is connected to sump 16 and is configured to convey the cleaning and disinfecting liquid from sump 16 to the plurality of orifices 4 and at least one connection port 25.

Thermal cleaning and disinfecting device 1 further comprises a heating system 6A for heating the cleaning and disinfecting liquid as well as to keep its temperature at a determined level. Heating system 6A comprises a steam generator 7, a heat transfer device 8A and a feeding pump 27 which is configured to convey liquid, in particular water, from source 22 to steam generator 7. Heat transfer device 8A is configured to receive steam generated by steam generator 7 and the cleaning and disinfecting liquid circulated by circulating pump 3. A mixing section 19 of the heat transfer device 8A comprises a mixing cavity in which the steam and the cleaning and disinfecting liquid contact one another and intermix, such that the steam condensates and transfers heat to the cleaning and disinfecting liquid. After mixing, the cleaning and disinfecting liquid having a higher temperature (compared to the temperature of the cleaning and disinfecting liquid entering the heat transfer device 8A) leaves heat transfer device 8A through an outlet 10. Outlet 10 connects to the plurality of orifices 4 so that the cleaning and disinfecting liquid heated by the steam is supplied to the plurality of orifices 4.

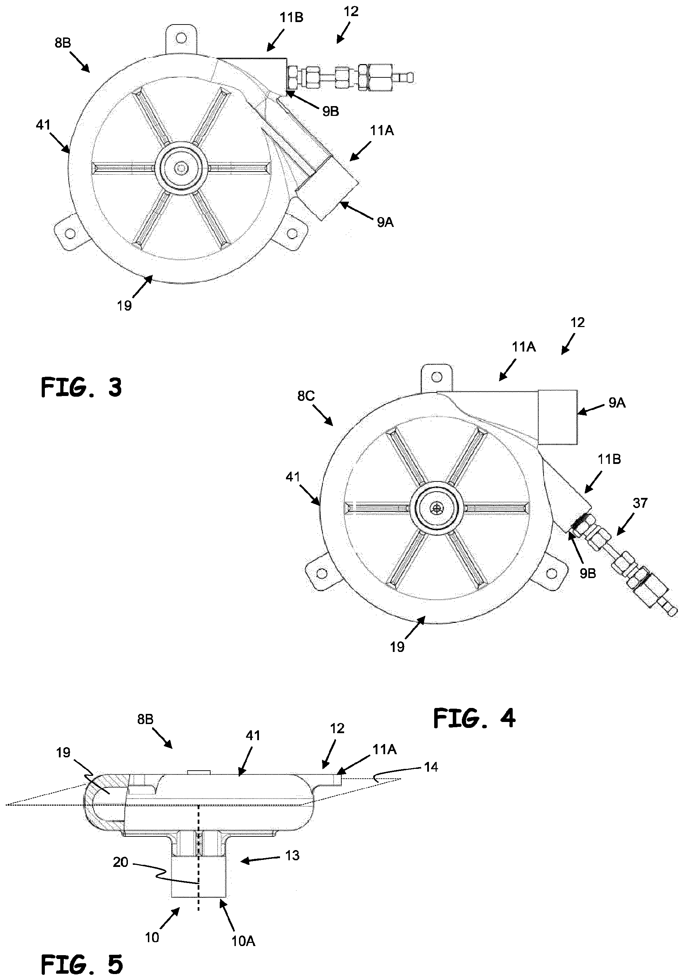

Control device 5 is configured to regulate and/ or control the operation of thermal cleaning and disinfecting device 1. Amongst others, control device 5 is configured to control the generation of steam and/ or the supply of steam to heat transfer device 8A (as well as to heat transfer devices 8B, 8C shown in Figures 3 - 5). According to the embodiment of Figure 1 control device 5 is therefore communicatively connected to feeding pump 27 and controls the feed rate of feeding pump 27 and thus the flow of liquid or water from source 22 to steam generator 7. The feed rate can be zero, so that no liquid or water is delivered to steam generator 7. By controlling the feed rate of feeding pump 27 control device 5 advantageously controls the generation of steam and the supply of steam, i.e., the amount of steam supplied, to heat transfer device 8A.

Further with reference to Figure 1, a temperature sensor 15 is provided at collecting tank 16 of washing chamber 2 to measure the temperature of the cleaning and disinfecting liquid at the bottom or in collecting tank 16 of washing chamber 2. Temperature sensor 15 is communicatively connected to control device 5, e.g., via control line 28. Temperature sensor 15 is configured to generate and send via signal line 28 a temperature signal indicative of the temperature of the cleaning and disinfecting liquid to control device 5. Based on the received temperature signal control device 5 controls the generation of steam and/ or supply of steam to heat transfer devices 8A, 8B, 8C by controlling feeding pump 27, in particular the feed rate of feeding pump 27, via control line 29.

Thermal cleaning and disinfecting device 1 further comprises a reservoir 30 for a cleaning or disinfecting agent. Reservoir 30 connects to washing chamber 2 via pipe 31, in particular to collecting tank 16, so that the cleaning or disinfecting agent can be added to the cleaning and disinfecting liquid in washing chamber 2. An agent actuator 32, e.g., an agent pump and/ or an agent valve as shown in Figure 1, is configured to the deliver the cleaning or disinfecting agent to washing chamber 2 and/ or to control the amount of the cleaning or disinfecting agent to be delivered. The agent actuator 32 is controllable by control device 5 via control line 33, so that the amount of added cleaning or disinfecting agent can be controlled and varied.

A sensor 17 for determining the concentration of the cleaning or disinfecting agent in the cleaning and disinfecting liquid is arranged at the bottom of the washing chamber 2, in particular at collecting tank 16. Sensor 17 is communicatively connected to control device 5, e.g., via control line 28. Sensor 17 for determining the concentration is configured to send a concentration signal indicative of the concentration of the cleaning or disinfecting agent in the cleaning and disinfecting liquid to control device 5. Based on the concentration signal control device 5 controls the supply of cleaning or disinfecting agent from reservoir 30 to the cleaning and disinfecting liquid through controlling agent actuator 32.

A sensor 34 for determining the filling level of the cleaning and disinfecting liquid in collecting tank 16 is arranged at the bottom of washing chamber 2. Sensor 34 communicatively connects to control device 5, e.g., via control line 28. Sensor 34 for determining the filling level is configured to send a filling level signal indicative of the filling level of the cleaning or disinfecting liquid in collecting tank 16 to control device 5. Based on the filling level signal control device 5 controls circulating pump 3 via control line 35.

Figures 2 - 5 show embodiments of heat transfer devices 8A, 8B, 8C which can be implemented in thermal cleaning and disinfecting device 1 as shown in Figure 1 or in similar thermal cleaning and disinfecting devices. Figure 2 in particular shows heat transfer device 8A of thermal cleaning and disinfecting device 1 of Figure 1.

Next, features which apply to all heat transfer devices 8A, 8B, 8C are discussed.

Heat transfer devices 8A, 8B, 8C comprise a first or feeding end 12 which connects to steam generator 7 and circulating pump 3, and a second or delivery end 13 which connects to orifices of the plurality of orifices 4. Heat transfer devices 8A, 8B, 8C comprise or are formed by a pipe 11 (see Figures 1 and 2) or are disk- or funnel-shaped (see Figures 3 - 5).

A first inlet 9A for receiving the cleaning and disinfecting liquid supplied by circulating pump 3 and a second inlet 9B for receiving the steam are provided at the first end 12 of the heat transfer devices 8A, 8B, 8C. First inlet 9A connects via pipe 36 to circulating pump 3, while second inlet 9B connects to steam generator 7 via pipe 37, see Figure 1. The first inlet 9A and the second inlet 9B are distinct inlets which are separated from one another. Pipe 11 of heat transfer devices 8A, 8B, 8C has a first branch 11A and a second branch 11B. First branch 11A connects to or comprises first inlet 9A and second branch 11B connects to or comprises second inlet 9B.

An outlet 10 is provided at second end 13 of pipe 11. Outlet 10 comprises a single outlet opening 10A which connects to the plurality of orifices 4 via pipe 38. Pipe 38 may branch into several sub-pipes which connect to orifices of the plurality of orifices 4 which are arranged on different locations, e.g., on different rotating arms 24.

Heat transfer devices 8A, 8B, 8C or pipe 11 comprise a mixing section 19. Mixing section 19 is downstream of first and second inlet 9A, 9B and upstream of outlet 10. Mixing section 19 is configured such that the steam and the cleaning and disinfecting liquid physically contact one another and intermix, in particular in an inner cavity of mixing section 19. Preferably, the length of pipe 11, in particular the length of the mixing section 19, is configured such, that the entire steam received by the heat transfer device 8A, 8B, 8C condensates within the heat transfer device 8A, 8B, 8C, so that no steam exits outlet 10 or enters pipe 38.

Next, features which do not apply to all heat transfer devices 8A, 8B, 8C, i.e., are specific to one or two of the heat transfer devices 8A, 8B, 8C, are discussed.

Heat transfer device 8A, shown in Figures 1 and 2, comprises a straight pipe 11 which extends along a longitudinal axis. Accordingly, first end 12 having at least one of the first inlet 9A or second inlet 9B and second end 13 having outlet 10 are opposite to one another. First branch 11A and second branch 11B form a Y or a Y-shaped first end of heat transfer device 8A.

Heat transfer device 8B, 8C, shown in Figures 3 - 5, comprise a disk- or funnel-shaped body 41. In particular body 41 is hollow and/ or comprises a cavity or receptacle which forms a mixing section 19. Mixing section 19 is configured such that it promotes the mixing of steam and the cleaning and disinfecting liquid due to the formation of a vortex of the steam and/ or cleaning and disinfecting liquid in its interior. Body 41 is arranged in a plane 14, wherein outlet 10 extends along an axis 20 which is disposed at an angle to plane 14, in particular approximately at 90°. The disk- or funnel-shaped heat transfer devices 8B, 8C have a very compact shape due to the radial or tangential arrangement of first and second branches 11A, 11B and the centrally or axially disposed outlet 10, which is advantageous when integrating the heat transfer devices into thermal cleaning and disinfecting device 1.

A difference between heat transfer device 8B of Figure 3 and 8C of Figure 4 is the arrangement of first branch 11A and/ or first inlet 9A and second branch 11B and/ or second inlet 9B, which take reversed positions.

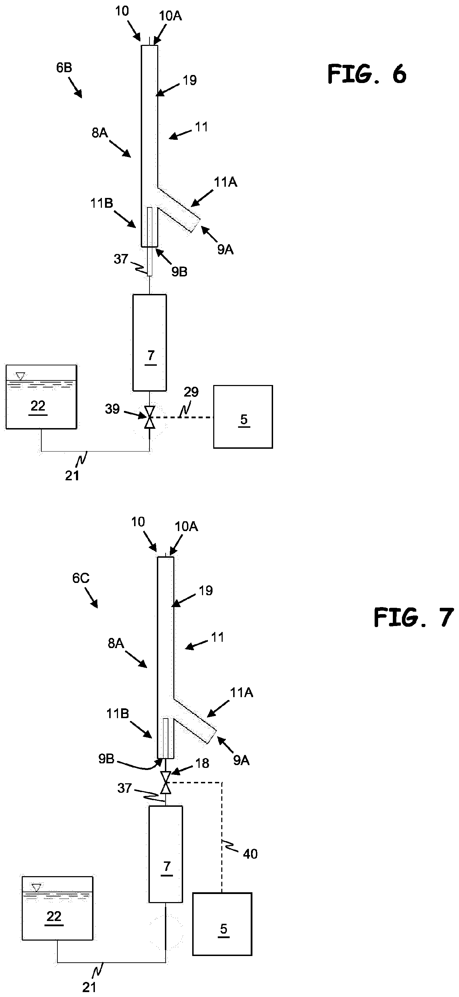

Figures 6 and 7 show alternative embodiments of heating systems 6B, 6C; in particular heating systems 6B, 6C have alternative configurations and/ or elements how control device 5 controls the generation of steam and/ or the supply of steam to heat transfer devices 8A, 8B, 8C. Heating systems 6B, 6C can for example be implemented in thermal cleaning and disinfecting device 1 shown in Figure 1. In order to avoid repetition only the differences between heating systems 6B, 6C and heating system 6A of Figure 1 are discussed in the following, while identical numerals in Figures 1, 6 and 7 comprise identical elements and functions.

According to Figure 6 a feeding valve 39 is arranged upstream of steam generator 7 and/ or downstream of liquid or water source 22. Feeding valve 39 may either replace feeding pump 27 of Figure 1 or be arranged in addition to feeding pump 27. Feeding valve 39 is configured to control the supply of liquid or water from the source 22 to steam generator 7. Feeding valve 39 is communicatively connected to and controlled by control device 5 via control line 29. Preferably, as described in the foregoing for feeding pump 27, based on a temperature signal provided by temperature sensor 15 control device 5 controls the generation of steam by steam generator 7 by controlling feeding valve 39, in particular the feed rate of liquid or water adjusted by feeding valve 39.

According to Figure 7 a control valve 18, also referred to as steam valve, is arranged downstream of steam generator 7 and/ or upstream of heat transfer device 8A, 8B, 8C. Steam valve 18 is controllable by control device 5 via control line 40. Preferably, based on a temperature signal provided by temperature sensor 15 control device 5 controls the supply or flow of steam from steam generator 7 to heat transfer devices 8A, 8B, 8C by controlling steam valve 18, in particular the feed rate of steam adjusted by valve 18.

The embodiments described or shown, in particular, serve to depict the invention.

## Claims

1. A thermal cleaning and disinfecting device (1) for cleaning and disinfecting medical, surgical, dental, veterinary as well as household items, comprising:
a washing chamber (2) for receiving the items to be cleaned and disinfected;
a circulating pump (3) for circulating a cleaning and disinfecting liquid;
a plurality of orifices (4) arranged in the washing chamber (2) for applying the cleaning and disinfecting liquid to the items to be cleaned and disinfected;
a control device (5); and
a heating system (6A, 6B, 6C) for heating the cleaning and disinfecting liquid, wherein
the heating system (6A, 6B, 6C) connects to the plurality of orifices (4), wherein
the heating system (6A, 6B, 6C) comprises a steam generator (7) and a heat transfer device (8A, 8B, 8C), wherein the heat transfer device (8A, 8B, 8C) is configured to receive steam generated by the steam generator (7) and the cleaning and disinfecting liquid circulated by the circulating pump (3), **characterized in that**
the heat transfer device (8A, 8B, 8C) comprises a mixing section (19) in which the steam and the cleaning and disinfecting liquid mix, such that the steam condensates and transfers heat to the cleaning and disinfecting liquid, and an outlet (10) which connects to the plurality of orifices (4) to supply the cleaning and disinfecting liquid to the plurality of orifices (4).

2. The thermal cleaning and disinfecting device (1) according to Claim 1, **characterized in that**
the heat transfer device (8A, 8B, 8C) comprises a first inlet (9A) for the cleaning and disinfecting liquid and a second inlet (9B) for the steam, wherein the first inlet (9A) and the second inlet (9B) are distinct inlets which are separated from one another.

3. The thermal cleaning and disinfecting device (1) according to Claim 1 or 2, **characterized in that**
the outlet (10) of the heat transfer device (8A, 8B, 8C) comprises a single outlet opening (10A) which connects to the plurality of orifices (4).

4. The thermal cleaning and disinfecting device (1) according to Claim 2 or 3, **characterized in that**
the heat transfer device (8A, 8B, 8C) comprises a pipe (11), which preferably has a first branch (11A) and a second branch (11B), wherein the first branch (11A) connects to the first inlet (9A) and the second branch (11B) connects to the second inlet (9B).

5. The thermal cleaning and disinfecting device (1) according to Claim 3 or 4, **characterized in that**
the heat transfer device (8A, 8B, 8C) comprises a straight pipe (11) with a first end (12) having at least one of the first inlet (9A) or second inlet (9B) and a second end (13) having the outlet (10) and which is opposite the first end (12).

6. The thermal cleaning and disinfecting device (1) according to any of the preceding Claims, **characterized in that**
the heat transfer device (8A, 8B, 8C), in particular the mixing section (19), comprises a disk-like shape or a funnel-like shape.

7. The thermal cleaning and disinfecting device (1) according to Claim 6, **characterized in that**
a body (41) of the heat transfer device (8A, 8B, 8C) having the disk or funnel-like shape is arranged in a plane (14), wherein the outlet (10) extends along an axis (20) which is disposed at an angle to the plane (14).

8. The thermal cleaning and disinfecting device (1) according to any of the preceding Claims, **characterized in that**
the control device (5) is configured to control the generation of steam and/ or the supply of steam to the heat transfer device (8A, 8B, 8C).

9. The thermal cleaning and disinfecting device (1) according to Claim 8, **characterized by**
a temperature sensor (15) which is configured to determine the temperature of the cleaning and disinfecting liquid and to send a temperature signal indicative of the temperature of the cleaning and disinfecting liquid to the control device (5), such that the control device (5) controls the generation of steam and/ or supply of steam to the heat transfer device (8A, 8B, 8C) based on the temperature signal indicative of the temperature of the cleaning and disinfecting liquid.

10. The thermal cleaning and disinfecting device (1) according to Claim 9, **characterized in that**
the temperature sensor (15) is arranged at a bottom, in particular at a collecting tank (16), of the washing chamber (2) to measure the temperature of the cleaning and disinfecting liquid at the bottom of the washing chamber (2).

11. The thermal cleaning and disinfecting device (1) according to any one of Claims 8 - 10, **characterized by**
a control valve (18) arranged between the steam generator (7) and the heat transfer device (8A, 8B, 8C) and controllable by the control device (5) to control the supply of steam to the heat transfer device (8A, 8B, 8C).

12. The thermal cleaning and disinfecting device (1) according to any of the preceding Claims, **characterized in that**
the control device (5) is configured to control the supply of a cleaning or disinfecting agent to the cleaning and disinfecting liquid.

13. The thermal cleaning and disinfecting device (1) according to Claim 12, **characterized by**
a sensor (17) for determining the concentration of the cleaning or disinfecting agent in the cleaning and disinfecting liquid and which is configured to send a concentration signal indicative of the concentration of the cleaning or disinfecting agent in the cleaning and disinfecting liquid to the control device (5), such that the control device (5) controls the supply of cleaning or disinfecting agent to the cleaning and disinfecting liquid based on the concentration signal.

14. The thermal cleaning and disinfecting device (1) according to Claim 13, **characterized in that**
the sensor (17) for determining the concentration is arranged at the bottom, in particular at the collecting tank (16), of the washing chamber (2) to measure the concentration of the cleaning or disinfecting agent in the cleaning and disinfecting liquid at the bottom of the washing chamber (2).

15. A method for heating a cleaning and disinfecting liquid in a thermal cleaning and
disinfecting device (1) according to any one of the Claims 1 - 14, **characterized by** providing a heating system (6A, 6B, 6C) comprising a steam generator (7) and a heat transfer device (8A, 8B, 8C), supplying steam generated by the steam generator (7) and cleaning and disinfecting liquid to the heat transfer device (8A, 8B, 8C),
mixing the steam and the cleaning and disinfecting liquid in a mixing section (19) of the heat transfer device (8A, 8B, 8C), such that the steam condensates and transfers heat to the cleaning and disinfecting liquid.

## Patentansprüche

1. Thermische Reinigungs- und Desinfektionsvorrichtung (1) zum Reinigen und Desinfizieren von medizinischen, chirurgischen, zahnmedizinischen, veterinärmedizinischen sowie Haushaltsgegenständen, umfassend:
eine Waschkammer (2) zur Aufnahme der zu reinigenden und zu desinfizierenden Gegenstände;
eine Umwälzpumpe (3) zum Umwälzen einer Reinigungs- und Desinfektionsflüssigkeit;
eine Vielzahl von in der Waschkammer (2) angeordneten Öffnungen (4) zum Aufbringen der Reinigungs- und Desinfektionsflüssigkeit auf die zu reinigenden und zu desinfizierenden Gegenstände;
eine Steuervorrichtung (5); und
ein Heizsystem (6A, 6B, 6C) zum Erhitzen der Reinigungs- und Desinfektionsflüssigkeit, wobei das Heizsystem (6A, 6B, 6C) mit der Vielzahl von Öffnungen (4) verbunden ist, wobei
das Heizsystem (6A, 6B, 6C) einen Dampferzeuger (7) und eine Wärmeübertragungsvorrichtung (8A, 8B, 8C) umfasst, wobei die Wärmeübertragungsvorrichtung (8A, 8B, 8C) so ausgebildet ist, dass sie den vom Dampferzeuger (7) erzeugten Dampf und die von der Umwälzpumpe (3) umgewälzte Reinigungs- und Desinfektionsflüssigkeit aufnimmt, **dadurch gekennzeichnet, dass** die Wärmeübertragungsvorrichtung (8A, 8B, 8C) einen Mischabschnitt (19) umfasst, in dem sich der Dampf und die Reinigungs- und Desinfektionsflüssigkeit vermischen, so dass der Dampf kondensiert und Wärme an die Reinigungs- und Desinfektionsflüssigkeit überträgt, sowie einen Auslass (10), der mit der Vielzahl von Öffnungen (4) verbunden ist, um die Reinigungs- und Desinfektionsflüssigkeit der Vielzahl von Öffnungen (4) zuzuführen.

2. Thermische Reinigungs- und Desinfektionsvorrichtung (1) nach Anspruch 1, **dadurch gekennzeichnet, dass**
die Wärmeübertragungsvorrichtung (8A, 8B, 8C) einen ersten Einlass (9A) für die Reinigungs- und Desinfektionsflüssigkeit und einen zweiten Einlass (9B) für den Dampf umfasst, wobei der erste Einlass (9A) und der zweite Einlass (9B) unterschiedliche Einlässe sind, die voneinander getrennt sind.

3. Thermische Reinigungs- und Desinfektionsvorrichtung (1) nach Anspruch 1 oder 2, **dadurch gekennzeichnet, dass**
der Auslass (10) der Wärmeübertragungsvorrichtung (8A, 8B, 8C) eine einzige Auslassöffnung (10A) umfasst, die mit der Vielzahl von Öffnungen (4) verbunden ist.

4. Thermische Reinigungs- und Desinfektionsvorrichtung (1) nach Anspruch 2 oder 3, **dadurch gekennzeichnet, dass**
die Wärmeübertragungsvorrichtung (8A, 8B, 8C) ein Rohr (11) umfasst, das vorzugsweise einen ersten Ast (11A) und einen zweiten Ast (11B) aufweist, wobei der erste Ast (11A) mit dem ersten Einlass (9A) und der zweite Ast (11B) mit dem zweiten Einlass (9B) verbunden ist.

5. Thermische Reinigungs- und Desinfektionsvorrichtung (1) nach Anspruch 3 oder 4, **dadurch gekennzeichnet, dass**
die Wärmeübertragungsvorrichtung (8A, 8B, 8C) ein gerades Rohr (11) mit einem ersten Ende (12), das zumindest den ersten Einlass (9A) oder den zweiten Einlass (9B) aufweist, und einem zweiten Ende (13), das den Auslass (10) aufweist und dem ersten Ende (12) gegenüberliegt, umfasst.

6. Thermische Reinigungs- und Desinfektionsvorrichtung (1) nach einem der vorstehenden Ansprüche, **dadurch gekennzeichnet, dass**
die Wärmeübertragungsvorrichtung (8A, 8B, 8C), insbesondere der Mischabschnitt (19), eine scheibenförmige oder trichterförmige Form aufweist.

7. Thermische Reinigungs- und Desinfektionsvorrichtung (1) nach Anspruch 6, **dadurch gekennzeichnet, dass**
ein Körper (41) der Wärmeübertragungsvorrichtung (8A, 8B, 8C) mit der scheibenförmigen oder trichterförmigen Form in einer Ebene (14) angeordnet ist, wobei sich der Auslass (10) entlang einer Achse (20) erstreckt, die gewinkelt zu der Ebene (14) angeordnet ist.

8. Thermische Reinigungs- und Desinfektionsvorrichtung (1) nach einem der vorstehenden Ansprüche, **dadurch gekennzeichnet, dass**
die Steuervorrichtung (5) ausgebildet ist, die Dampferzeugung und/ oder die Zufuhr von Dampf zur Wärmeübertragungsvorrichtung (8A, 8B, 8C) zu steuern.

9. Thermische Reinigungs- und Desinfektionsvorrichtung (1) nach Anspruch 8, **gekennzeichnet durch**
einen Temperatursensor (15), der ausgebildet ist, die Temperatur der Reinigungs- und Desinfektionsflüssigkeit zu bestimmen und ein Temperatursignal, das die Temperatur der Reinigungs- und Desinfektionsflüssigkeit wiedergibt, an die Steuervorrichtung (5) zu senden, so dass die Steuervorrichtung (5) die Dampferzeugung und/ oder die Zufuhr von Dampf zu der Wärmeübertragungsvorrichtung (8A, 8B, 8C) basierend auf dem Temperatursignal, das die Temperatur der Reinigungs- und Desinfektionsflüssigkeit wiedergibt, steuert.

10. Thermische Reinigungs- und Desinfektionsvorrichtung (1) nach Anspruch 9, **dadurch gekennzeichnet, dass**
der Temperatursensor (15) am Boden, insbesondere an einem Sammelbehälter (16), der Waschkammer (2) angeordnet ist, um die Temperatur der Reinigungs- und Desinfektionsflüssigkeit am Boden der Waschkammer (2) zu messen.

11. Thermische Reinigungs- und Desinfektionsvorrichtung (1) nach einem der Ansprüche 8 - 10, **gekennzeichnet durch**
ein zwischen dem Dampferzeuger (7) und der Wärmeübertragungsvorrichtung (8A, 8B, 8C) angeordnetes Steuerventil (18), das durch die Steuervorrichtung (5) steuerbar ist, um die Zufuhr von Dampf zu der Wärmeübertragungsvorrichtung (8A, 8B, 8C) zu steuern.

12. Thermische Reinigungs- und Desinfektionsvorrichtung (1) nach einem der vorstehenden Ansprüche, **dadurch gekennzeichnet, dass**
die Steuervorrichtung (5) ausgebildet ist, die Zufuhr eines Reinigungs- oder Desinfektionsmittels zu der Reinigungs- und Desinfektionsflüssigkeit zu steuern.

13. Thermische Reinigungs- und Desinfektionsvorrichtung (1) nach Anspruch 12, **gekennzeichnet durch**
einen Sensor (17) zur Bestimmung der Konzentration des Reinigungs- oder Desinfektionsmittels in der Reinigungs- und Desinfektionsflüssigkeit, der ausgebildet ist, ein Konzentrationssignal, das die Konzentration des Reinigungs- oder Desinfektionsmittels in der Reinigungs- und Desinfektionsflüssigkeit angibt, an die Steuervorrichtung (5) zu senden, so dass die Steuervorrichtung (5) die Zufuhr von Reinigungs- oder Desinfektionsmittel zu der Reinigungs- und Desinfektionsflüssigkeit basierend auf dem Konzentrationssignal steuert.

14. Thermische Reinigungs- und Desinfektionsvorrichtung (1) nach Anspruch 13, **dadurch gekennzeichnet, dass**
der Sensor (17) zur Bestimmung der Konzentration am Boden, insbesondere am Sammelbehälter (16), der Waschkammer (2) angeordnet ist, um die Konzentration des Reinigungs- oder Desinfektionsmittels in der Reinigungs- und Desinfektionsflüssigkeit am Boden der Waschkammer (2) zu messen.

15. Verfahren zum Erhitzen einer Reinigungs- und Desinfektionsflüssigkeit in einer thermischen Reinigungs- und Desinfektionsvorrichtung (1) nach einem der Ansprüche 1 -14, **gekennzeichnet durch**
Bereitstellen eines Heizsystems (6A, 6B, 6C) mit einem Dampferzeuger (7) und einer Wärmeübertragungsvorrichtung (8A, 8B, 8C), Zuführen von Dampf, der von dem Dampferzeuger (7) erzeugt ist, und Reinigungs- und Desinfektionsflüssigkeit zu der Wärmeübertragungsvorrichtung (8A, 8B, 8C), Mischen des Dampfes und der Reinigungs- und Desinfektionsflüssigkeit in einem Mischabschnitt (19) der Wärmeübertragungsvorrichtung (8A, 8B, 8C), so dass der Dampf kondensiert und Wärme an die Reinigungs- und Desinfektionsflüssigkeit überträgt.

## Revendications

1. Dispositif de nettoyage et de désinfection thermique (1) destiné au nettoyage et à la désinfection d'articles médicaux, chirurgicaux, dentaires, vétérinaires et ménagers, comprenant:
une chambre de lavage (2) pour recevoir les articles à nettoyer et à désinfecter;
une pompe de circulation (3) pour faire circuler un liquide de nettoyage et de désinfection;
une pluralité d'orifices (4) disposés dans la chambre de lavage (2) pour appliquer le liquide de nettoyage et de désinfection sur les articles à nettoyer et à désinfecter;
un dispositif de commande (5); et
un système de chauffage (6A, 6B, 6C) destiné au chauffage du liquide de nettoyage et de désinfection, dans lequel le système de chauffage (6A, 6B, 6C) est relié à la pluralité d'orifices (4), dans lequel
le système de chauffage (6A, 6B, 6C) comprend un générateur de vapeur (7) et un dispositif de transfert de chaleur (8A, 8B, 8C), dans lequel le dispositif de transfert de chaleur (8A, 8B, 8C) est conçu pour recevoir la vapeur produite par le générateur de vapeur (7) et le liquide de nettoyage et de désinfection mis en circulation par la pompe de circulation (3), **caractérisé en ce que**
le dispositif de transfert de chaleur (8A, 8B, 8C) comprend une section de mélange (19) dans laquelle la vapeur et le liquide de nettoyage et de désinfection se mélangent, de sorte que la vapeur se condense et transfère de la chaleur au liquide de nettoyage et de désinfection et une sortie (10) qui est reliée à la pluralité d'orifices (4) pour alimenter la pluralité d'orifices (4) en liquide de nettoyage et de désinfection.

2. Dispositif de nettoyage et de désinfection thermique (1) selon la revendication 1, **caractérisé en ce que**
le dispositif de transfert de chaleur (8A, 8B, 8C) comprend une première entrée (9A) pour le liquide de nettoyage et de désinfection et une deuxième entrée (9B) pour la vapeur, dans lequel la première entrée (9A) et la deuxième entrée (9B) sont distinctes et séparées une de l'autre.

3. Dispositif de nettoyage et de désinfection thermique (1) selon la revendication 1 ou 2, **caractérisé en ce que**
la sortie (10) du dispositif de transfert de chaleur (8A, 8B, 8C) comprend un unique orifice de sortie (10A) qui est relié à la pluralité d'orifices (4).

4. Dispositif de nettoyage et de désinfection thermique (1) selon la revendication 2 ou 3, **caractérisé en ce que**
le dispositif de transfert de chaleur (8A, 8B, 8C) comprend un tube (11), qui comporte de préférence une première branche (11A) et une deuxième branche (11B), dans lequel la première branche (11A) est raccordée à la première entrée (9A) et la deuxième branche (11B) est raccordée à la deuxième entrée (9B).

5. Dispositif de nettoyage et de désinfection thermique (1) selon la revendication 3 ou 4, **caractérisé en ce que**
le dispositif de transfert de chaleur (8A, 8B, 8C) comprend un tube droit (11) présentant une première extrémité (12) munie d'au moins une première entrée (9A) ou une deuxième entrée (9B), et une deuxième extrémité (13) munie de la sortie (10) et opposée à la première extrémité (12).

6. Dispositif de nettoyage et de désinfection thermique (1) selon une quelconque des revendications précédentes, **caractérisé en ce que**
le dispositif de transfert de chaleur (8A, 8B, 8C), notamment la section de mélange (19), présente une forme de disque ou d'entonnoir.

7. Dispositif de nettoyage et de désinfection thermique (1) selon la revendication 6, **caractérisé en ce que**
un corps (41) du dispositif de transfert de chaleur (8A, 8B, 8C) en forme de disque ou d'entonnoir est disposé dans un plan (14), dans lequel la sortie (10) s'étend le long d'un axe (20) qui forme un angle par rapport au plan (14).

8. Dispositif de nettoyage et de désinfection thermique (1) selon une quelconque des revendications précédentes, **caractérisé en ce que**
le dispositif de commande (5) est configuré pour commander la production et/ou l'alimentation en vapeur du dispositif de transfert de chaleur (8A, 8B, 8C).

9. Dispositif de nettoyage et de désinfection thermique (1) selon la revendication 8, **caractérisé par**:
un capteur de température (15), qui est configuré pour déterminer la température du liquide de nettoyage et de désinfection et pour envoyer un signal de température indicatif de la température du liquide de nettoyage et de désinfection au dispositif de commande (5), de sorte que le dispositif de commande (5) commande la production et/ou l'alimentation en vapeur du dispositif de transfert de chaleur (8A, 8B, 8C) sur la base du signal de température indicatif de la température du liquide de nettoyage et de désinfection.

10. Dispositif de nettoyage et de désinfection thermique (1) selon la revendication 9, **caractérisé en ce que**:
le capteur de température (15) est disposé au fond, notamment au niveau d'un réservoir de collecte (16), de la chambre de lavage (2) afin de mesurer la température du liquide de nettoyage et de désinfection au fond de la chambre de lavage (2).

11. Dispositif de nettoyage et de désinfection thermique (1) selon une quelconque des revendications 8 à 10, **caractérisé par**:
une vanne de commande (18) disposée entre le générateur de vapeur (7) et le dispositif de transfert de chaleur (8A, 8B, 8C), qui peut être commandée par le dispositif de commande (5) pour commander l'alimentation en vapeur du dispositif de transfert de chaleur (8A, 8B, 8C).

12. Dispositif de nettoyage et de désinfection thermique (1) selon une quelconque des revendications précédentes, **caractérisé en ce que**:
le dispositif de commande (5) est configuré pour commander l'alimentation en agent de nettoyage ou de désinfection vers le liquide de nettoyage et de désinfection.

13. Dispositif de nettoyage et de désinfection thermique (1) selon la revendication 12, **caractérisé par**:
un capteur (17) destiné à déterminer la concentration de l'agent de nettoyage ou de désinfection dans le liquide de nettoyage et de désinfection et qui est configuré pour envoyer un signal de concentration indicatif de la concentration de l'agent de nettoyage ou de désinfection dans le liquide de nettoyage et de désinfection au dispositif de commande (5), de sorte que le dispositif de commande (5) commande l'alimentation en agent de nettoyage ou de désinfection vers liquide de nettoyage et de désinfection sur la base du signal de concentration.

14. Dispositif de nettoyage et de désinfection thermique (1) selon la revendication 13, **caractérisé en ce que**:
le capteur (17) destiné à déterminer la concentration est disposé au fond, notamment au niveau du réservoir de collecte (16), de la chambre de lavage (2) afin de mesurer la concentration de l'agent de nettoyage ou de désinfection dans le liquide de nettoyage et de désinfection au fond de la chambre de lavage (2).

15. Procédé de chauffage d'un liquide de nettoyage et de désinfection dans un dispositif de nettoyage et de désinfection thermique (1) selon une quelconque des revendications 1 à 14, **caractérisé par**:
la fourniture d'un système de chauffage (6A, 6B, 6C) comprenant un générateur de vapeur (7) et un dispositif de transfert de chaleur (8A, 8B, 8C), l'alimentation du dispositif de transfert de chaleur (8A, 8B, 8C) en vapeur générée par le générateur de vapeur (7) et en liquide de nettoyage et de désinfection;
le mélange de la vapeur et du liquide de nettoyage et de désinfection dans une section de mélange (19) du dispositif de transfert de chaleur (8A, 8B, 8C), de sorte que la vapeur se condense et transfère sa chaleur au liquide de nettoyage et de désinfection.
